# EUROPEAN PATENT SPECIFICATION

(11) **EP 1 668 027 B1**
(45) Date of publication and mention of the grant of the patent: **19.05.2010**
(21) Application number: 04787113.2
(22) Date of filing: 09.09.2004
(51) Int. Cl.: C07K 7/64

(54) **CYCLIC PEPTIDES ACTING AS UROTENSIN-II ANTAGONISTS**
CYCLISCHE PEPTIDE, DIE ALS UROTENSIN-II-ANTAGONISTEN WIRKEN
PEPTIDES CYCLIQUES ANTAGONISTES DE L'UROTENSINE-II

(30) Priority: 11.09.2003 IT FI20030238
(43) Date of publication of application: 14.06.2006
(73) Proprietor: Novellino, Ettore, 83040 Montemarano (IT); Grieco, Paolo, 80121 Napoli (IT); Rovero, Paolo, 50127 Firenze (IT)
(72) Inventor: Novellino, Ettore, 83040 Montemarano (IT); Grieco, Paolo, 80121 Napoli (IT); Rovero, Paolo, 50127 Firenze (IT)
(74) Representative: Gervasi, Gemma
(86) International application number: PCT/EP2004/052101
(87) International publication number: WO 2005/023845

(56) References cited:
- GRIECO, PAOLO ET AL: "A New, Potent Urotensin II Receptor Peptide Agonist Containing a Pen Residue at the Disulfide Bridge" JOURNAL OF MEDICINAL CHEMISTRY , 45(20), 4391-4394 CODEN: JMCMAR; ISSN: 0022-2623, 26 September 2002 (2002-09-26), XP002320555 cited in the application
- CAMPIGLIA P ET AL: "P 326: Development of new urotensin-II receptor ligands." BIOPOLYMERS, vol. 71, no. 3, 23 June 2003 (2003-06-23), page 374, XP002320556 & 18TH AMERICAN PEPTIDE SYMPOSIUM ON PEPTIDE REVOLUTION: GENOMICS, PROTEOMICS AND THERAPEUTICS; BOSTON, MA, USA; JULY 19-23, 2003 ISSN: 0006-3525
- GRIECO P ET AL: "Design, synthesis, conformational analysis, and biological studies of urotensin-II lactam analogues" BIOORGANIC & MEDICINAL CHEMISTRY, ELSEVIER SCIENCE LTD, GB, vol. 10, no. 12, 20 December 2002 (2002-12-20), pages 3731-3739, XP002234088 ISSN: 0968-0896
- PATACCHINI, RICCARDO ET AL: "Urantide: An ultrapotent urotensin II antagonist peptide in the rat aorta" BRITISH JOURNAL OF PHARMACOLOGY, CODEN: BJPCBM; ISSN: 0007-1188, vol. 140, no. 7, December 2003 (2003-12), pages 1155-1158, XP002320557
- DARLAK, K. ET AL: "Facile preparation of disulfide-bridged peptides using the polymer-supported oxidant CLEAR-OX" JOURNAL OF PEPTIDE RESEARCH, CODEN: JPERFA; ISSN: 1397-002X, vol. 63, no. 3, March 2004 (2004-03), pages 303-312, XP002320507

### SEQUENCE LISTING

<110> Novellino, Ettore
   Grieco, Paolo
   Rovero, Paolo
<120> Cyclic peptides acting as urotensin-II antagonists
<130> 4501PTWO
<150> FI2003A000238
   <151> 2003-09-11
<160> 11
<170> PatentIn version 3.1
<210> 1
   <211> 8
   <212> PRT
   <213> Artificial sequence
<220>
   <223> Cyclic peptide
<220>
   <221> MISC_FEATURE
   <222> (2)..(2)
   <223> X is penicillamine
<220>
   <221> DISULFID
   <222> (2)..(7)
   <223> cyclic disulfide
<220>
   <221> MISC_FEATURE
   <222> (4)..(4)
   <223> residue having D configuration
<220>
   <221> MISC_FEATURE
   <222> (5)..(5)
   <223> X is Ornitine
<400> 1
   Asp Xaa Phe Trp Xaa Tyr Cys Val
   1 5
<210> 2
   <211> 7
   <212> PRT
   <213> Artificial sequence
<220>
   <223> Cyclic peptide
<220>
   <221> MISC_FEATURE
   <222> (1)..(1)
   <223> X is penicillamine
<220>
   <221> MISC_FEATURE
   <222> (3)..(3)
   <223> residue having D configuration
<220>
   <221> DISULFID
   <222> (1)..(6)
   <223> cyclic disulfide
<220>
   <221> MOD_RES
   <222> (1)..(1)
   <223> ACETYLATION
<220>
   <221> MISC_FEATURE
   <222> (4)..(4)
   <223> X is Ornitine
<400> 2
   Xaa Phe Trp Xaa Tyr Cys Val
   1 5
<210> 3
   <211> 11
   <212> PRT
   <213> Artificial sequence
<220>
   <223> Cyclic peptide
<220>
   <221> MISC_FEATURE
   <222> (5)..(5)
   <223> X is Penicillamine
<220>
   <221> MISC_FEATURE
   <222> (8)..(8)
   <223> X is Ornitine
<220>
   <221> DISULFID
   <222> (5)..(10)
   <223> cyclic disulfide
<220>
   <221> MOD_RES
   <222> (7)..(7)
   <223> ACETYLATION
<400> 3
   Glu Thr Pro Asp Xaa Phe Trp Xaa Tyr Cys Val
   1               5                   10
<210> 4
   <211> 8
   <212> PRT
   <213> Artificial sequence
<220>
   <223> cyclic peptide
<220>
   <221> DISULFID
   <222> (2).. (7)
   <223> cyclic disulfide
<220>
   <221> MISC_FEATURE
   <222> (2).. (2)
   <223> X is Penicillamine
<220>
   <221> MISC_FEATURE
   <222> (5)..(5)
   <223> X is Ornitine
<220>
   <221> MISC_FEATURE
   <222> (4)..(4)
   <223> residue having D configuration
<400> 4
   Asp Xaa Phe Trp Xaa Tyr Cys Val
   1               5
<210> 5
   <211> 8
   <212> PRT
   <213> Artificial sequence
<220>
   <223> cyclic peptide
<220>
   <221> DISULFID
   <222> (2)..(7)
   <223> cyclic disulfide
<220>
   <221> MISC_FEATURE
   <222> (2)..(2)
   <223> X is Penicillamine
<220>
   <221> MISC_FEATURE
   <222> (5)..(5)
   <223> X is Ornitine
<220>
   <221> MISC_FEATURE
   <222> (4)..(4)
   <223> residue having D configuration
<400> 5
   Asp Xaa Phe Trp Xaa Tyr Cys Val
   1               5
<210> 6
   <211> 8
   <212> PRT
   <213> Artificial sequence
<220>
   <223> cyclic peptide
<220>
   <221> DISULFID
   <222> (2)..(7)
   <223> cyclic sulfide
<220>
   <221> MISC_FEATURE
   <222> (2)..(2)
   <223> X is Penicillamine
<220>
   <221> MISC_FEATURE
   <222> (4)..(4)
   <223> residue having D configuration
<400> 6
   Asp Xaa Phe Trp Lys Tyr Cys Val
   1 5
<210> 7
   <211> 8
   <212> PRT
   <213> Artificial sequence
<220>
   <223> cyclic peptide
<220>
   <221> DISULFID
   <222> (2)..(7)
   <223> cyclic disulfide
<220>
   <221> MISC_FEATURE
   <222> (2)..(2)
   <223> X is Penicillamine
<220>
   <221> MISC_FEATURE
   <222> (4)..(4)
   <223> X is 3-(1-naphtyl)alanine
<220>
   <221> MISC_FEATURE
   <222> (5)..(5)
   <223> X is Ornitine
<220>
   <221> MISC_FEATURE
   <222> (4)..(4)
   <223> residue having D configuration
<400> 7
   Asp Xaa Phe Xaa Xaa Tyr Cys Val
   1 5
<210> 8
   <211> 8
   <212> PRT
   <213> Artificial sequence
<220>
   <223> cyclic peptide
<220>
   <221> DISULFID
   <222> (2)..(7)
   <223> cyclic disulfide
<220>
   <221> MISC_FEATURE
   <222> (4)..(4)
   <223> residue having D configuration
<220>
   <221> MISC_FEATURE
   <222> (2)..(2)
   <223> X is Penicillamine
<220>
   <221> MISC_FEATURE
   <222> (4)..(4)
   <223> X is 3-(2-naphtyl)alanine
<220>
   <221> MISC_FEATURE
   <222> (5)..(5)
   <223> X is Ornitine
<400> 8
   Asp Xaa Phe Xaa Xaa Tyr Cys Val
   1 5
<210> 9
   <211> 8
   <212> PRT
   <213> Artificial sequence
<220>
   <223> cyclic peptide
<220>
   <221> DISULFID
   <222> (2)..(7)
   <223> cyclic disulfide
<220>
   <221> MISC_FEATURE
   <222> (2)..(2)
   <223> X is Penicillamine
<220>
   <221> MISC_FEATURE
   <222> (5)..(5)
   <223> X is Ornitine
<220>
   <221> MISC_FEATURE
   <222> (4)..(4)
   <223> residue having D configuration
<400> 9
   Asp Xaa Phe Trp Xaa Tyr Cys Val
   1 5
<210> 10
   <211> 8
   <212> PRT
   <213> Artificial sequence
<220>
   <223> cyclic peptide
<220>
   <221> DISULFID
   <222> (2)..(7)
   <223> cyclic disulfide
<220>
   <221> MISC_FEATURE
   <222> (2)..(2)
   <223> X is Penicillamine
<220>
   <221> MISC_FEATURE
   <222> (5)..(5)
   <223> X is Ornitine
<220>
   <221> MISC_FEATURE
   <222> (4)..(4)
   <223> residue having D configuration
<400> 10
   Asp Xaa Phe Trp Xaa Tyr Cys Val
   1 5
<210> 11
   <211> 8
   <212> PRT
   <213> Artificial sequence
<220>
   <223> cyclic peptide
<220>
   <221> DISULFID
   <222> (2)..(7)
   <223> cyclic disulfide
<220>
   <221> MISC_FEATURE
   <222> (2)..(2)
   <223> X is Penicillamine
<220>
   <221> MISC_FEATURE
   <222> (5)..(5)
   <223> X is Ornitine
<220>
   <221> MISC_FEATURE
   <222> (4) ..(4)
   <223> residue having D configuration
<400> 11
   Asp Xaa Phe Trp Xaa Tyr Cys Val
   1               5

## Description

### FIELD OF THE INVENTION

The present invention refers to cyclic peptides analogues of Urotensin-II having the formula (I) reported herein below, useful for the preparation of pharmaceutical compositions for the treatment of diseases associated with Urotensin-II imbalance.

### STATE OF THE ART

Urotensin-II is a cyclic peptide originally isolated from teleost fish urophysis and sequenced more than 20 years ago (Pearson, D. et al. Proc. Natl. Acad. Sci. USA (1980), 77, 5021-5024). Different structural forms of Urotensin-II were subsequently described in various species of fish and amphibians, as well as in mammals, including humans. In 1999 the Urotensin-II receptor was identified (Ames R.S. et al. Nature (1999), 401, 282-286). Several publications indicate that

Urotensin-II is a potent constrictor of certain isolated human artery and veins, *in vitro*. Moreover, Urotensin-II induces contraction of non-vascular smooth muscle of cardiac and respiratory systems.

Urotensin-II and its receptor were identified in human cardiac tissues and it was reported that Urotensin-II exerts a strong inotropic effects in the human heart *in vitro*. Taken together, data reported in the literature indicate that Urotensin-II modulates cardiovascular homeostasis and therefore may play a key role in certain cardiovascular pathologies (Ames R.S. et al. Nature (1999), 401, 282-286).

Consequently, synthetic analogues of Urotensin-II can be used to develop agonists, antagonists, and inhibitors of Urotensin-II and then for the pharmacological treatment of pathological states associated with Urotensin-II balance. For this purpose, cyclic peptides that are Urotensin-II analogues and comprise exclusively amino acid residues not containing sulphur, were developed and described in the International Patent Application No. WO 01/37856 (SmithKline Beecham Corp.).

Other cyclic peptides that are Urotensin-II analogues and have the above mentioned pharmacological use, were also described in the International Patent Application No. WO 01/37780 (SmithKline Beecham Corp.); in this case the peptides contain two cysteine residues linked by a disulphide bridge.

Cyclic peptide analogues of Urotensin-II having formula X-cyclo[M-A_{w}-B-C-D_{z}-N]-Y were described in Grieco P. et al., J. Med. Chem., 2002, 45:4391-4394; in these cyclic peptides a disulphide bridge is formed between the side chains of two amino acid residues containing sulphur M and N, which are never simultaneously cystein; moreover, in the above said formula (I) w and z are 0 or 1, X is chosen among H, Ac, H-Asp, Ac-Asp, and H-Glu-Thr-Pro-Asp; Y is chosen among OH, NH₂, Val-OH, e Val-NH₂; A and D are chosen among Phe, D-Phe, Tyr, D-Tyr, Pro, D-Pro, Nal(1), D-Nal(1), Nal(2), D-Nal(2), Cha, Chg, and Bpa; B is selected from Trp, D-Trp, e (α-Me)Trp; and C is selected from Lys, Arg, Orn, e Dab.

Campiglia P. et al., Biopolymers, 2003, 71(3), 374, P326 describes cyclic peptide analogues of Urotensin-II having formula H-Asp-cyclo[Pen-Phe-Trp-Xaa-Xaa-Cys]-Val-OH, wherein Xaa are selected from several unconventional amino acids in order to examine the importance of these positions on interaction with Urotensin II receptor.

Cyclic peptide analogues of Urotensin-II having formula H-Asp-cyclo[Xaa-Phe-Trp-Lys-Tyr-Yaa]-Val-OH, wherein Xaa is Cys, Dap Orn or Lys and Yaa is Cys, Asp or Glu were described in Grieco P. et al., Bioorg. & Med Chem., 2002, 10:3731-3739; these derivatives were tested for contractile activity on the rat thoracic aorta.

The above mentioned Urotensin-11 peptide analogues described to date in the literature, although endowed with a certain antagonist activity toward Urotensin-II receptor, nevertheless show limited specificity and sometimes a residual agonist activity which severely limits their pharmacological and therapeutic usefulness.

Therefore it is still felt the need of peptides endowed with antagonist activity toward the Urotensin-II receptor without the drawbacks reported above for the known peptides above reported, thus being useful for an effective treatment of the above mentioned pathological conditions.

### SUMMARY OF THE INVENTION

Now the Applicants have surprisingly found that cyclic peptides of formula (I) reported below possess antagonist activity and high specificity toward human Urotensin-II receptor, and are devoid of any residual agonist activity.

Thanks to these properties the novel peptides of formula (I) are superior in efficacy as compared to the previously reported Urotensin-II peptide inhibitors, because they show a very potent antagonist activity and are devoid of the above mentioned limitations, typical of the previously described peptides. Therefore, the present peptides of formula (I) can be successfully used for the treatment of pathological conditions associated with Urotensin-II imbalance in humans, such as hypertension, cardiac arrhythmia, heart stroke, angina, ischemia of myocardium and restenosis.

Subject of the present invention are therefore cyclic peptides of general formula (I)

X cyclo[Pen-A-B-C-D-Cys]-Y (I)

wherein:
X is selected from the group consisting of H, Ac, H-Asp, Ac-Asp, H-Phe, H-D-Phe and H-Glu-Thr-Pro-Asp,
Y is selected from the group consisting of OH, NH₂, Val-OH, and Val-NH₂,
A and D, equal or different from each other, are selected from the group consisting of Phe, D-Phe, Tyr and D-Tyr,
B is selected from the group consisting of D-Trp, D-(α-Me)Trp, D-Trp(Me), D-Trp(CHO), D-Nal(1) and D-Nal(2);
C is selected from the group consisting of Lys, Asp, Glu, Orn, Cit, Dap and (p-amino)Phe;
   or pharmaceutically acceptable salts thereof.

Further subjects of the invention are the pharmaceutical compositions comprising the above said cyclic peptides of formula (I) and/or their salts.

Further subject of the invention is the use of the cyclic peptides of formula (I) and/or of their salts as reagents for the pharmacological characterization of

Urotensin-II receptors, and their use for the preparation of pharmaceutical compositions for the treatment of pathological conditions associated with unbalance of Urotensin-II.

Features and advantages of the present invention will be illustrated in details in the following description.

### DETAILED DESCRIPTION OF THE INVENTION

Within the scope of the present invention natural amino acids are indicated using the common three-letters code, while not genetically coded amino acids were indicated using the following abbreviations:
- Pen: Penicillamine;
- Nal(1): 3-(1-Naphtyl)alanine;
- Nal(2): 3-(2-Naphtyl)alanine;
- Orn: Ornitine;
- Cit: Citrulline;
- Dap: 2-amino-propionic acid.
With the abbreviation Ac, an acetyl group is meant.

Some of the peptides of formula (I) according to the present invention are included in the general formula already described in Grieco P. et al., J. Med. Chem., 2002, 45:4391-4394, but they are not specifically described.

The present peptides contain a disulphide bridge between Pen and Cys residues and are characterized by the presence of an amino acid residue B of the D series and by a suitable combination of amino acid residues B and C as reported above.

In fact, the Applicants surprisingly found that whenever the two central residues B and C within the cyclic portion of the said peptides of general formula (I) are those mentioned above, and the B residue presents the inversion of configuration from L to D, the resulting peptides are endowed with potent and highly specific antagonist activity toward the human Urotensin-II receptor, and are devoid of any residual agonist activity toward the same receptor.

Preferred peptides of formula (I) according to the invention are those in which C is selected from the group consisting of Lys, Orn, and (p-amino)Phe.

Particularly notable results were observed when in the general formula (I) B is D-Trp and C is Orn.

According to a preferred embodiment of the present invention in the general formula (I) X is H-Asp, Y is Val-OH, B is D-Trp, and C is Orn.

Particularly preferred are the following peptides:
H-Asp-cyclo[Pen-Phe-DTrp-Orn-Tyr-Cys]-Val-OH (SEQ. ID. NO. 1)
Ac-cyclo[Pen-Phe-DTrp-Orn-Tyr-Cys]-NH₂ (SEQ. ID. NO. 2)
H-Glu-Thr-Pro-Asp-cyclo[Pen-Phe-DTrp-Orn-Tyr-Cys]-Val-OH (SEQ. ID. NO. 3)
H-Asp-cyclo[Pen-DPhe-DTrp-Orn-Tyr-Cys]-Val-OH (SEQ. ID. NO. 4)
H-Asp-cyclo[Pen-Phe-DTrp-Orn-DTyr-Cys]-Val-OH (SEQ. ID. NO. 5)
H-Asp-cyclo[Pen-Phe-DTrp-Lys-Tyr-Cys]-Val-OH (SEQ. ID. NO. 6)
H-Asp-cyclo[Pen-Phe-DNal(1)-Orn-Tyr-Cys]-Val-OH (SEQ. ID. NO. 7)
H-Asp-cyclo[Pen-Phe-DNal(2)-Orn-Tyr-Cys]-Val-OH (SEQ. ID. NO. 8)
H-Asp-cyclo[Pen-Phe-D-(α-Me)Trp-Orn-Tyr-Cys]-Val-OH (SEQ. ID. NO. 9)
H-Asp-cyclo[Pen-Phe-D-Trp(Me)-Orn-Tyr-Cys]-Val-OH (SEQ. ID. NO. 10)
H-Asp-cyclo[Pen-Phe-D-Trp(CHO)-Orn-Tyr-Cys]-Val-OH (SEQ. ID. NO. 11).

The amino acid residues included in said peptides of formula (I) belong to the L-series, unless otherwise specified.

The peptides according to the invention can be prepared by conventional solution and solid-phase peptide synthesis, known to any person skilled in the art. Thus, for example, to prepare peptides with a free acid at C-terminal, the synthesis in solid-phase can be performed using a Wang resin. Instead, to obtain peptides as C-terminal amide the synthesis in solid phase can be performed using a resin like as a PAL resin [Tris(alkoxy)benzylamide].

The synthesis of the peptides object of this application can be performed by using orthogonal protection using Fluorenylmethoxycarbonyl (hereinafter referred to as "Fmoc") and t-Butyloxycarbonyl (hereinafter referred to as "Boc") as protecting groups at α-amino group of amino acids, and t-Butyl (hereinafter referred to as "t- Bu) and Benzyl (hereinafter referred to as "Bzl") as protecting groups at side-chain of amino acids, such as Asp, Glu (as t-butyl ester), Lys (Boc), Tyr, Thr (OtBu).

The thiolic group of Cys and Pen can be protected by a Trityl group (hereinafter referred to as "Trt").

Regarding the strategy "Fmoc/t-Bu", the peptide synthesis consists of the following steps:
a) loading of first C-terminal residue onto the resin;
b) deprotection of α-amino group by a solution of piperidine 25% (v/v) in dimethylformamide (DMF);
c) coupling of the next amino acid, appropriately protected as above said, using a condensing or activating agent, such as N,N'-dicyclohexylcarbodiimide (DCC), O-(benzotriazole-1-yl)-1,1,3,3-tetramethyluronium hexafluorophosphate (HBTU), O-benzotriazole-1-yl-1,1,3,3-tetramethyluronium tetrafluoroborate (TBTU), 1-hydroxy-benzotriazole (HOBt), etc.;
d) repeating of steps a) and b) until the desired sequence is completed;
e) cleavage of the linear peptide from the resin and deprotection of amino acids side-chains by using trifluoroacetic acid in the presence of suitable "scavengers", such as triethylsilane (TES) and water (5% v/v, each);
f) formation of disulfide bridge by oxidation, for example with Ferricyanide, oxygen or dimethyl sulfoxide (DMSO);
g) purification of crude cyclic peptide by semipreparative HPLC, preferably by reverse-phase HPLC on C-18 column, using as eluents acetonitrile and water in the presence of 0.1 % of trifluoroacetic acid (TFA);
h) lyophilization of final purified product;
i) characterization of final compound by analytical HPLC, Mass spectrometry (MS) and amino acids analysis (AAA).

The present peptides of formula (I), in free form or as pharmaceutically acceptable salts, can be used for the preparation of pharmaceutical compositions according to conventional methods of preparation, well-known in pharmaceutical field.

These pharmaceutical compositions can be conventionally formulated, and may further comprise one or more pharmaceutically acceptable excipients and/or diluents.

The administration of the present compositions is feasible by any conventional way, for example by parenteral injection in the form of injectable solutions or suspensions, or by oral, topic, nasal administration, etc.

The formulations of the present peptides include compresses, capsules, pills, solutions, dispersions, creams and ointments, emulsions, aerosol, and can also be used to accomplish a controlled or delayed release of the active compound.

The present pharmaceutical compositions may comprise the peptides of formula (I) according to the invention as sole active component, or may comprise the present peptides in combination with other active compounds or adjuvants, suitably chosen according to the pathological conditions to be treated.

The pharmaceutical compositions comprising the present peptides are useful for the pharmacological treatment of pathologies associated with an alteration of

Urotensin-II balance, such as hypertension, heart arrhythmia, heart stroke, angina, ischemia of myocardium and restenosis.

The present peptides of general formula (I) can also be used as reagents in biotechnological and pharmaceutical research, for example as reagents in characterisation of human Urotensin-II receptor.

All peptides according to the invention were synthesised by the methodologies described above and illustrated in details in Example 1.

The following examples are reported for illustrative but not limitative purposes of present invention.

### EXAMPLE1

### Preparation of H-Asp-cvclo[Pen-Phe-D-Trp-Orn-Tvr-Cvs]-Val-OH (SEQ. ID. NO. 1)

The compound was synthesised by standard solid-phase methodology, starting from 0.5 g of Wang resin (0.7 mmol/g) using *N*^{α}-Fmoc chemistry and an orthogonal side chain protection strategy. The first amino acid, *N*^{α}-Fmoc-Val-OH, was linked to the resin using as coupling reagents a 3-fold excess of HBTU/HOBt in the presence of N,N'-diisopropylethylamine (DIEA). The following amino acids were then added stepwise to the growing peptide: N^{α}-Fmoc-Cys(Trt)-OH, N^{α}-Fmoc-Tyr(OtBu)-OH, N^{α}-Fmoc-Orn(N^{ε}-Boc)-OH, N^{α}-Fmoc-D-Trp(Nⁱⁿ-Boc)-OH, N^{α}-Fmoc-Phe-OH, N^{α}-Fmoc-Pen(Trt)-OH and N^{α}-Fmoc-Asp(OtBu)-OH using standard solid phase methods. Each coupling reaction was achieved using a 3-fold excess of amino acid and of HBTU/HOBt in presence of DIEA. The *N*^{α}-Fmoc protecting group was removed by treating the protected peptide resin with 25% piperidine solution in DMF (1 x 50 mL, 5 min, 1 x 50 mL, 20 min). The peptide resin was washed with DMF (3 x 50 mL), DCM (3 x 50 mL) and again with DMF.

Upon complete formation of the protected linear peptide, the compound was cleaved from the resin and the other side chain protecting groups removed using the following mixture: TFA/TES/H₂O (9:0.5:0.5) for 3 h. All procedures were done under an Argon atmosphere. The resin was removed from solution by filtration and the crude peptide was recovered by precipitation with cold anhydrous ethyl ether giving a white powder that was purified by preparative HPLC on a C18-bonded silica gel column (Vydac 218TP1010, 1.0 x 25 cm) eluted with a linear gradient of acetonitrile in aqueous 0.1% of TFA (v/v). The purification was monitored at 280 nm, and the fraction corresponding to the major peak were collected, combined, and lyophilised to give the final compound as a pure (>98%) white powder. The linear peptide was characterised by analytical HPLC and FAB-MS (m/e 1073.282 [M⁺]). The final cyclic peptide, containing the disulfide bridge, was achieved by oxidation with an aqueous solution of potassium ferricyanide at pH = 8.5 (ammonium buffer). This step was monitored by analytical HPLC to evaluate the grade of conversion. Final cyclic peptide was purified by reverse-phase HPLC using the condition above described.

### EXAMPLES 2 - 9

Following the same synthetic methodology reported above and using appropriate reagents and amino acids, the following peptides 2-9 were also synthesised:

| Example | Peptide | **MS (m/e)** |
|---|---|---|
| 2 | Ac-cyclo[Pen-Phe-DTrp-Orn-Tyr-Cys]-NH₂ (SEQ. ID. NO. 2) | 900.112 |
| 3 | H-Glu-Thr-Pro-Asp-cyclo[Pen-Phe-DTrp-Orn-Tyr-Cys]-Val-OH (SEQ. ID. NO. 3) | 1400.622 |
| 4 | H-Asp-cyclo[Pen-DPhe-DTrp-Orn-Tyr-Cys]-Val-OH (SEQ. ID. NO. 4) | 1073.282 |
| 5 | H-Asp-cyclo[Pen-Phe-DTrp-Orn-DTyr-Cys]-Val-OH (SEQ. ID. NO. 5) | 1073.282 |
| 6 | H-Asp-cyclo[Pen-Phe-DTrp-Lys-Tyr-Cys]-Val-OH (SEQ. ID. NO. 6) | 1087.309 |
| 7 | H-Asp-cyclo[Pen-Phe-DNal(1)-Orn-Tyr-Cys]-Val-OH (SEQ. ID. NO. 7) | 1083.298 |
| 8 | H-Asp-cyclo[Pen-Phe-DNal(2)-Orn-Tyr-Cys]-Val-OH (SEQ. ID. NO. 8) | 1083.298 |
| 9 | H-Asp-cyclo[Pen-Phe-D(a-Me)Trp-Orn-Tyr-Cys]-Val-OH (SEQ. 10. NO. 9) | 1087.282 |
| 10 | H-Asp-cyclo[Pen-Phe-DTrp(Me)-Orn-Tyr-Cys]-Val-OH (SEQ. ID. NO. 10) | 1087.282 |
| 11 | H-Asp-cyclo[Pen-Phe-DTrp(CHO)-Orn-Tyr-Cys]-Val-OH (SEQ. ID. NO. 11) | 1101.298 |

### BIOLOGICAL ACTIVITY

The cyclic peptides of the invention were tested in binding and functional assays as reported below.

### Binding Assays

All experiments were performed on membranes obtained from stable CHO-K1 cells expressing the recombinant human UT receptor. The radioligand used for competition experiments was [¹²⁵I]Urotensin II. Nonspecific binding was determined in the presence of 1 mM of unlabelled hU-II. In this binding assay human Urotensin-II showed an affinity of Kᵢ = 1.8 nM (nanomolar), while the compound achieved as in Example 1 and tested in same conditions described above, showed an affinity value expressed as pKi = 8.3.

### Functional Assay

The biological activity of the said peptides of formula (I) was evaluated using the rat isolated thoracic aorta assay (Camarda, V. et al. Naunyn-Schmiedeberg's Arch. Pharmacol. (2002) 365, 141). The thoracic aorta was excised from male albino rats (Wistar) and cleared of surrounding tissue; from each vessel, a helically cut strip was prepared, and then it was cut into two parallel strips. The endothelium was removed and the preparations were placed in 5 ml organ baths filled with oxygenated normal Krebs-Henseleit solution. Motor activity of the strips was recorded isotonically (load 5 mN). A cumulative concentration-response curve to hU-II was constructed on one of the two strips, which served as control.

The other strip received the antagonist peptide under examination and, after a 30-min incubation period, hU-II was administered cumulatively. Antagonist activity was expressed in terms of pKB (negative logarithm of the antagonist dissociation constant; Kenankin, T.P. Pharmacologic analysis of drugreceptor interaction, third ed. Lippincott-Raven, Philadelphia, PA, 1997).

The peptide of formula (I), prepared as described in Example 1, and tested in these conditions, showed to be a potent antagonist at human Urotensin-II receptor, with a pKb value of 8.3. This value is almost 70 fold higher as compared to other antagonist compounds at Urotensin-II receptor described to date in literature.

## Claims

1. Cyclic peptides of general formula (I):
X-cyclo[Pen-A-B-C-D-Cys]-Y (I)
Wherein:
X is selected from the group consisting of H, Ac, H-Asp, Ac-Asp, H-Phe, H-D-Phe and H-Glu-Thr-Pro-Asp,
Y is selected from the group consisting of OH, NH₂, Val-OH, and Val-NH₂,
A and D, equal or different from each other, are selected from the group consisting of Phe, D-Phe, Tyr and D-Tyr,
B is selected from the group consisting of D-Trp, D-(α-Me)Trp, D-Trp(Me), D-Trp(CHO), D-Nal(1) and D-Nal(2);
C is selected from the group consisting of Lys, Asp, Glu, Orn, Cit, Dap and (p-amino)Phe;
or pharmaceutically acceptable salts thereof.

2. Cyclic peptides of formula (I) according to claim 1, wherein C is selected from the group consisting of Lys, Orn, and (p-ammino)Phe.

3. Cyclic peptides of formula (I) according to claim 1, wherein B is D-Trp and C is Orn.

4. Cyclic peptides of formula (I) according to claim 1, wherein X is H-Asp, Y is Val-OH, B is D-Trp, and C is Orn.

5. Cyclic peptides of formula (I) according to claim 1, selected from the following compounds:
H-Asp-cyclo[Pen-Phe-DTrp-Orn-Tyr-Cys]-Val-OH (SEQ. ID. NO. 1)
Ac-cyclo[Pen-Phe-DTrp-Orn-Tyr-Cys]-NH₂ (SEQ. ID. NO. 2)
H-Glu-Thr-Pro-Asp-cyclo[Pen-Phe-DTrp-Orn-Tyr-Cys]-Val-OH (SEQ. ID. NO. 3)
H-Asp-cyclo[Pen-DPhe-DTrp-Orn-Tyr-Cys]-Val-OH (SEQ. ID. NO. 4)
H-Asp-cyclo[Pen-Phe-DTrp-Orn-DTyr-Cys]-Val-OH (SEQ. ID. NO. 5)
H-Asp-cyclo[Pen-Phe-DTrp-Lys-Tyr-Cys]-Val-OH (SEQ. ID. NO. 6)
H-Asp-cyclo[Pen-Phe-DNal(1)-Orn-Tyr-Cys]-Val-OH (SEQ. ID. NO. 7)
H-Asp-cyclo[Pen-Phe-DNal(2)-Orn-Tyr-Cys]-Val-OH (SEQ. ID. NO. 8)
H-Asp-cyclo[Pen-Phe-D-(α-Me)Trp-Orn-Tyr-Cys]-Val-OH (SEQ. ID. NO. 9)
H-Asp-cyclo[Pen-Phe-D-Trp(Me)-Orn-Tyr-Cys]-Val-OH (SEQ. ID. NO. 10)
H-Asp-cyclo[Pen-Phe-D-Trp(CHO)-Orn-Tyr-Cys]-Val-OH (SEQ. ID. NO. 11).

6. A reagent for the pharmacological characterisation of human Urotensin-II receptor comprising at least a cyclic peptide of general formula (I) as described in claims 1-5, or a pharmaceutically acceptable salt thereof.

7. A pharmaceutical composition comprising as active compound at least a cyclic peptide of general formula (I) as described in claims 1-5, or a pharmaceutically acceptable salt thereof, optionally further comprising pharmaceutically acceptable excipients and/or diluents.

8. Use of cyclic peptides of general formula (I) as described in claims 1-5, for the preparation of pharmaceutical compositions useful for the treatment of pathological conditions associated with unbalance of Urotensin-II.

9. Use of cyclic peptides of formula (I) according to claim 8, wherein said pathological conditions are selected from the group consisting of hypertension, cardiac arrhythmia, heart stroke, angina, ischemia of myocardium and restenosis.

10. Use of cyclic peptides of general formula (I) as described in claims 1-5, as reagents for the pharmacological characterization of human Urotensin-II receptor.

## Patentansprüche

1. Cyclische Peptide der allgemeinen Formel (I):
X-cyclo[Pen-A-B-C-D-Cys]-Y (I)
In welcher:
X aus der Gruppe bestehend aus H, Ac, H-Asp, Ac-Asp, H-Phe, H-D-Phe und H-Glu-Thr-Pro-Asp ausgewählt wird,
Y aus der Gruppe bestehend aus OH, NH₂, Val-OH und Val-NH₂ ausgewählt wird,
A und D, identisch oder voneinander verschieden, aus der Gruppe bestehend aus Phe, D-Phe, Tyr und D-Tyr ausgewählt werden,
B aus der Gruppe bestehend aus D-Trp, D-(α-Me)Trp, D-Trp(Me), D-Trp(CHO), D-Nal(1) und D-Nal(2) ausgewählt wird,
C aus der Gruppe bestehend aus Lys, Asp, Glu, Orn, Cit, Dap und (p-Amino)Phe ausgewählt wird;
oder pharmazeutisch zulässige Salze davon.

2. Cyclische Peptide der Formel (I) gemäß Anspruch 1, wobei C aus der Gruppe bestehend aus Lys, Orn und (p-Amino)Phe ausgewählt wird.

3. Cyclische Peptide der Formel (I) gemäß Anspruch 1, wobei B D-Trp ist und C Orn ist.

4. Cyclische Peptide der Formel (I) gemäß Anspruch 1, wobei X H-Asp, ist, Y Val-OH ist, B D-Trp ist und C Orn ist.

5. Cyclische Peptide der Formel (I) gemäß Anspruch 1, ausgewählt aus den folgenden Verbindungen:
H-Asp-cyclo[Pen-Phe-DTrp-Orn-Tyr-Cys]-Val-OH (SEQ. ID. NO. 1)
Ac-cyclo[Pen-Phe-DTrp-Orn-Tyr-Cys]-NH₂ (SEQ. ID. NO. 2)
H-Glu-Thr-Pro-Asp-cyclo[Pen-Phe-DTrp-Orn-Tyr-Cys]-Val-OH (SEQ. ID. NO. 3)
H-Asp-cyclo[Pen-DPhe-DTrp-Orn-Tyr-Cys]-Val-OH (SEQ. ID. NO. 4)
H-Asp-cyclo[Pen-Phe-DTrp-Orn-DTyr-Cys]-Val-OH (SEQ. ID. NO. 5)
H-Asp-cyclo[Pen-Phe-DTrp-Lys-Tyr-Cys]-Val-OH (SEQ. ID. NO. 6)
H-Asp-cyclo[Pen-Phe-DNal(1)-Orn-Tyr-Cys]-Val-OH (SEQ. ID. NO. 7)
H-Asp-cyclo[Pen-Phe-DNal(2)-Orn-Tyr-Cys]-Val-OH (SEQ. ID. NO. 8)
H-Asp-cyclo[Pen-Phe-D-(α-Me)Trp-Orn-Tyr-Cys]-Val-OH (SEQ. ID. NO. 9)
H-Asp-cyclo[Pen-Phe-D-Trp(Me)-Orn-Tyr-Cys]-Val-OH (SEQ. ID. NO. 10)
H-Asp-cyclo[Pen-Phe-D-Trp(CHO)-Orn-Tyr-Cys]-Val-OH (SEQ. ID. NO. 11).

6. Reagenz für die pharmakologische Charakterisierung des humanen Urotensin-II-Rezeptors, umfassend mindestens ein cyclisches Peptid der allgemeinen Formel (I) gemäß den Ansprüchen 1-5 oder ein pharmazeutisch zulässiges Salz davon.

7. Pharmazeutische Zusammensetzungen, enthaltend mindestens ein cyclisches Peptid der allgemeinen Formel (I) gemäß den Ansprüchen 1-5 oder ein pharmazeutisch zulässiges Salz davon als den wirksamen Bestandteil, und gegebenenfalls weiterhin enthaltend pharmazeutisch zulässige Hilfsstoffe und/oder Verdünnungsmittel.

8. Verwendung von cyclischen Peptiden der allgemeinen Formel (I) gemäß den Ansprüchen 1-5 zur Herstellung von pharmazeutischen Zusammensetzungen, die zur Behandlung von mit einem Urotensin-II-Ungleichgewicht einhergehenden pathologischen Zuständen von Nutzen sind.

9. Verwendung von cyclischen Peptiden der Formel (I) gemäß Anspruch 8, wobei genannte pathologische Zustände aus der Gruppe bestehend aus Hypertonie, Herzrhythmusstörungen, Herzinfarkt, Angina, Ischämie des Myokards und Restenose ausgewählt werden.

10. Verwendung von cyclischen Peptiden der allgemeinen Formel (I) gemäß den Ansprüchen 1-5 als Reagenzien für die pharmakologische Charakterisierung des humanen Urotensin-II-Rezeptors.

## Revendications

1. Peptides cycliques de formule générale (I) :
X-cyclo[Pen-A-B-C-O-Cys]-Y (I)
dans laquelle :
X est choisi dans le groupe constitué de H, Ac, H-Asp, Ac-Asp, H-Phe, H-D-Phe et H-Glu-Thr-Pro-Asp,
Y est choisi dans le groupe constitué d'OH, NH₂, Val-OH, et Val-NH₂,
A et D, égaux ou différents l'un de l'autre, sont choisis dans le groupe constitué de Phe, D-Phe, Tyr et D-Tyr,
B est choisi dans le groupe constitué de D-Trp, D-(α-Me)Trp, D-Trp(Me), D-Trp(CHO), D-Nal(1) et D-Nal(2) ;
C est choisi dans le groupe constitué de Lys, Asp, Glu, Orn, Cit, Dap et (p-amino)Phe ;
ou leurs sels pharmaceutiquement acceptables.

2. Peptides cycliques de formule (I) selon la revendication 1, dans lesquels C est choisi dans le groupe constitué de Lys, Orn, et (p-amino)Phe.

3. Peptides cycliques de formule (I) selon la revendication 1, dans lesquels B est un D-Trp et C est un Orn.

4. Peptides cycliques de formule (I) selon la revendication 1, dans lesquels X est un H-Asp, Y est un Val-OH, B est un D-Trp, et C est un Orn.

5. Peptides cycliques de formule (I) selon la revendication 1, choisis parmi les composés suivants :
H-Asp-cyclo[Pen-Phe-DTrp-Orn-Tyr-Cys]-Val-OH (SEQ. ID. NO. 1)
Ac-cyclo[Pen-Phe-DTrp-Orn-Tyr-Cys]-NH₂ (SEQ. ID. NO. 2)
H-Glu-Thr-Pro-Asp-cyclo[Pen-Phe-DTrp-Orn-Tyr-Cys]-Val-OH (SEQ. ID. NO. 3)
H-Asp-cyclo[Pen-DPhe-DTrp-Orn-Tr-Cys]-Val-On (SEQ. ID. NO. 4)
H-Asp-cyclo[Pen-Phe-DTrp-Orn-Dtyr-Cys]-Val-OH (SEQ. ID, NO. 5)
H-Asp-cyclo[Pen-Phe-DTrp-Lys-Tyr-Cys]-Val-OH (SEQ. ID. NO. 6)
H-Asp-cyclo[Pen-Phe-Dnal(1)-Orn-Tyr-Cys]-Val-OH (SEQ. ID. NO. 7)
H-Asp-cyclo[Pen-Phe-DNal(2)-Orn-Tyr-Cys]-Val-OH (SEQ. ID. NO. 6)
H-Asp-cyclo[Pen-Phe-D-(α-Me)Trp-Orn-Tyr-Cys]-Val-OH (SEQ_{.} ID. NO. 9)
H-Asp-cylco[Pen-Phe-D-Trp(Me)-Orn-Tyr-Cys]-Val-OH (SEQ. ID. NO. 10)
H-Asp-cyclo[Pen-Phe-D-TrP(CHO)-Orn-Tyr-Cys]-Val-OH (SEQ. ID. NO. 11).

6. Réactif pour la caractérisation pharmacologique du récepteur d'urotensine-II humain comprenant au moins un peptide cyclique de formule générale (I) comme décrit dans les revendications 1-5, ou son sel pharmaceutiquement acceptable.

7. Composition pharmaceutique comprenant comme composé actif au moins un peptide cyclique de formule générale (I), comme décrit dans les revendications 1-5,
ou son sel pharmaceutiquement acceptable, comprenant éventuellement également des excipients et/ou diluants pharmaceutiquement acceptables.

8. Utilisation de peptides cycliques de formule générale (I) comme décrit dans les revendications 1-5, pour la préparation de compositions pharmaceutiques utiles pour le traitement de conditions pathologiques associées au déséquilibre d'Urotensine-II.

9. Utilisation de peptides cycliques de formule (I) selon la revendication 8, dans laquelle lesdites conditions pathologiques sont choisies dans le groupe constitué d'hypertension, arythmie cardiaque, crise cardiaque, angine de poitrine, ischémie du myocarde et resténose.

10. Utilisation de peptides cycliques de formule générale (I) comme décrit dans les revendications 1-5, comme réactifs pour la caractérisation pharmacologique du récepteur d'urotensine II humain.
